# EUROPEAN PATENT SPECIFICATION

(11) **EP 3 612 243 B1**
(45) Date of publication and mention of the grant of the patent: **20.08.2025**
(21) Application number: 18724043.7
(22) Date of filing: 23.04.2018
(51) Int. Cl.: A61M 1/00, A61F 13/00

(54) **WOUND DRAIN**
WUNDDRAINAGE
DRAIN DE PLAIE

(30) Priority: 22.04.2017 US 201762488743 P
(43) Date of publication of application: 26.02.2020
(73) Proprietor: Phase One Health, LLC, Nashville, TN 37228-1403 (US)
(72) Inventor: RODEWALD, Albert, Nashville TN 37228-1403 (US); PORTER, Robert Stephen, Nashville TN 37228-1403 (US); ARMOCAST, John, Nashville TN 37228-1403 (US)
(74) Representative: Lucas, Phillip Brian
(86) International application number: PCT/US2018/028928
(87) International publication number: WO 2018/195549

(56) References cited:
- DE-A1- 102014 227 041
- FR-A- 1 163 907
- US-A1- 2001 029 956
- US-A1- 2009 234 307

## Description

### TECHNICAL FIELD

The present disclosure relates to a wound drain for use in wound care treatment, particularly negative pressure wound treatment.

### CROSS-REFERENCE TO RELATED APPLICATIONS

This application claims benefit of the following U.S. Provisional Patent Application No. 62/488,743 filed on April 22, 2017.

### BACKGROUND

Chronic and acute wounds, including pressure ulcers, diabetic wounds, and bums, present significant challenges to the health care industry. Patient care providers are actively seeking methods, devices, and systems for treating such wounds at a lower cost and with greater efficacy.

Conventional treatments for chronic wounds frequently include negative pressure therapy and/or hyperbaric oxygen therapy.

Negative pressure therapy is the controlled application of sub-atmospheric pressure to a wound using a therapy unit, such as a vacuum or suction device, to expose the wound to negative pressure to help promote wound healing. The wound is typically covered to facilitate the application of negative pressure and suction at the wound area. Various types of resilient, open cell foam surface dressings are typically sealed within an adhesive drape to provide the sub-atmospheric pressure at the wound site. Exudates are drained from the wound site and typically directed to a canister that stores the fluids and/or infectious material until properly disposed. Negative pressure wound therapy is often prescribed for chronic and acute wound types such as diabetic wounds, pressure ulcers, abdominal wounds, trauma wounds, various bums, flaps and grafts. However, negative pressure therapy may be less effective on patients having vascular disorders, such as diabetes, because negative pressure therapy can create a hypoxic environment at the wound. In current hospital settings, portable vacuum pumps are often rented or purchased for the purpose of providing negative pressure therapy. This can significantly increase the cumulative costs of providing wound care.

Hyperbaric oxygen therapy is the controlled application of oxygen to a wound at greater-than-atmospheric pressure(s). Oxygen is typically required for all new cell growth. Chronic or non-healing wounds tend to exhibit low oxygen tensions, or tend to be ischemic. A wound can become dormant if the amount of poorly oxygenated wound tissue reaches a critical mass. In this state, the body may no longer recognize the need to heal the affected area, thereby exacerbating the lack of oxygen in the wound and impairing healing of the wound by the body. Oxygen therapy is particularly useful for patients with poor circulation. The oxygen helps to kill bacteria and when applied to an open wound at a hyperbaric level, the oxygen is dissolved into the wound and absorbed by the surface wound tissue. The cells of the wound tissue that absorb the oxygen will begin metabolic activity in response to the increased oxygen tension. When the oxygen source is removed, the previously active cells request more oxygen from the body. The body responds by beginning to form new blood cells, and thus, starting the healing process. Accordingly, when delivered to a wound site under hyperbaric conditions, oxygen may act as a primary wound treatment fluid

More recently, wound care systems and methods have been developed that combine negative and positive pressure (e.g. hyperbaric oxygen) wound treatment therapies. Such methods and systems are described, for example, in U.S. Pat. No. 7,648,488, issued Jan. 19, 2010; U.S. Pat. No. 8,357,130, issued Jan. 22, 2013; U.S. Patent Publication No. 2008/0140029; and U.S. Patent Publication No. 2010/0121287. Documents US2009/234307 A1, FR1163907 A, US2001/029956 A1 and DE102014227041 A1 disclose different wound drains.

Nevertheless, there is a need for wound drains that are particularly suited for negative pressure therapy or combined negative and positive pressure therapy. More particularly, there is a need for wound drains that have a structure that prevents collapse under negative pressure and avoids ingesting of foreign materials during therapy. The present disclosure addresses these needs.

### BRIEF SUMMARY OF THE INVENTION

The invention is set out in the appended set of claims.

### BRIEF DESCRIPTION OF THE SEVERAL VIEWS OF THE DRAWINGS

FIG. 1 is a perspective view of an embodiment of the wound drain.
FIG. 2 is another perspective view of the wound drain of Fig. 1 showing internal features in shadow.
FIG. 3 is a side sectional view of the wound drain of Fig. 1.
FIG. 4 is an exploded perspective view of another embodiment of the wound drain of this invention.
FIG. 5 is a side sectional view of a third embodiment of the wound drain of this invention.
FIG. 6A depicts a side view of another embodiment of the wound drain. FIG. 6B is a cross sectional view of the wound drain depicted in FIG. 6A.
FIGS. 7A to 7C depict several views of an embodiment of a wound drain having a dual lumen configuration.
FIGS. 8A and 8B depict a grate useful in the wound drains of the present disclosure.
FIGS 9 A and 9B depict yet another embodiment of a wound drain according to the present disclosure.

### DETAILED DESCRIPTION OF THE INVENTION

The wound drain of the present disclosure advantageously incorporates structural components that prevent the drain from collapsing under negative pressure and from ingesting foreign materials introduced into the wound cavity, such as gauze and sponges, which can obstruct exudate flow through the drain. The wound drains are used in a conventional manner, being placed over wound sites and secured by an occlusive dressing hermetically sealing the wound site. The wound drains can be employed, for example, whenever a controlled distribution and/or collection of fluid(s) is desired. The wound drains facilitate negative pressure wound treatment, but may in other embodiments be adapted for use with combined negative and positive pressure (atmospheric or above) wound treatment.

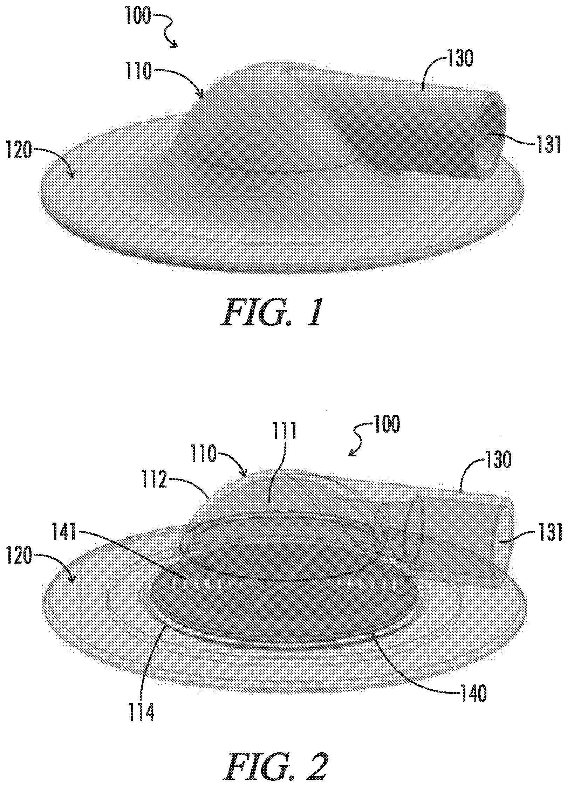

Referring now to the drawings, FIGS. 1-3 illustrate an embodiment of the wound drain, which is designated generally as reference numeral 100. Wound drain 100 is configured to have a central semi-spherical dome 110 integrally rising from a flat annular flange 120 extending radially from the dome. Dome 110 has a dome wall 112 defining an interior dome chamber 111 with an open bottom. Flange 120 transitions radially outward from dome wall 112 at the bottom of dome 110. Dome 110 also has an integral annular lip 114 that extends inward from dome wall 112 at the bottom of dome chamber 111 near the transition to flange 120. Dome 110 may also has a plurality of radial support ribs (not shown) extending into dome chamber 111.

Dome 110 also has a port connection 130 integrally formed therewith. Port connection 130 defines a fluid passage 131 in open communication with the dome chamber 111. Port connection 130 is configured to receive one or more fluid lines or lumens (not shown). The lumens are operatively connected to the negative pressure supply and/or drain line so that the negative pressure draws exudate into fluid passage 131 through dome interior 111.

Wound drain 100 is formed, molded or otherwise made of a pliable material, suitable for surgical and medical use. The pliable material may be a material suitable for medical purposes, such as a silicon rubber or Arkema Pebax^{®} polymer. The pliable construction allows the drain to conform and contort so that the flange lies flat against the patient's skin, even over, around and into body creases and folds.

Wound drain 100 also includes a structural support or screen 140. As shown, screen 140 is configured as a flat circular disc having a plurality of concentric arcuate openings or slits 141 formed in a segmented quadrant pattern. In other embodiments, the openings or slits in the screen may take other shapes and configurations. Slits 141 allow exudate and other fluids to pass from the wound cavity into dome chamber 111, while preventing gauze and sponges from being ingested into the dome. The openings or slits are configured and dimensioned to provide sufficient area to allow fluid flow across the bottom of dome 110, but are small enough to prevent foreign materials from migrating into and clogging fluid passage 131 of port connection 130.

Screen 140 is restrictively seated at the bottom of dome 100 between an annular lip 114 and dome wall 112. The conjunction between lip 114 and dome wall 112 creates a screen seat 116 which holds screen 140 at the bottom of dome chamber 111 in a "press-fit" type connection. In certain embodiments, suitable adhesives may be added at screen seat 116 to further join and secure screen 140 within dome 110. Screen 140 is typically formed, molded or otherwise made of a pliable material, suitable for surgical and medical use, but also more rigid than the material of dome 110 and flange 120. The screen material is selected so that screen 140 when seated within dome 110 provides sufficient structural integrity to hold the dome wall upright and prevent dome wall 112 from deforming and collapsing under negative pressure. The dome shape has a generous radius to allow for more pliability and reduced pressure points on the wound.

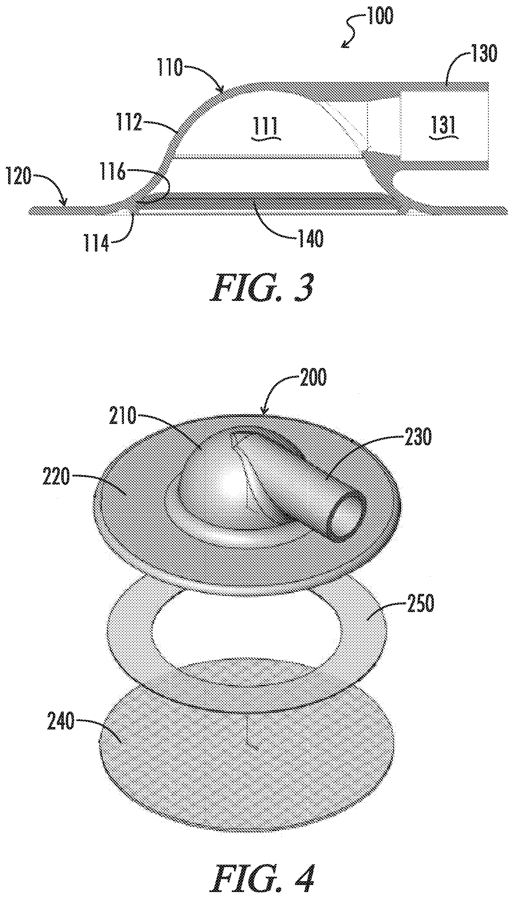

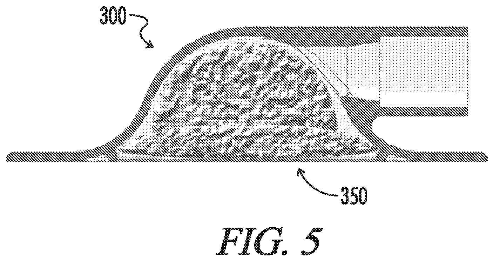

FIGS. 4 and 5 illustrate two alternative embodiments of the wound drain, identified respectively as reference numerals 200 and 300. Each wound drain 200 and 300 is identical in configuration and composition of drain 100 above, except that they employ a different structural component for preventing the drain from collapsing under negative pressure and from ingesting foreign materials introduced into the wound cavity, such as gauze and sponges, both of which can obstruct exudate flow through the drain. Wound drain 200 includes a dome 210, port connection 230 and a flat screen 240 that is affixed to the bottom of flange 220 covering substantially the entire surface area of drain 200. As with screen 140 above, screen 240 is typically formed, molded or otherwise made of a pliable material, suitable for surgical and medical use. More importantly, screen 240 is made of a material that is more rigid than the material of the dome and flange of drain 200. Screen 240 is affixed to the bottom surface of flange 220 by adhesive layer 250. Turning to FIG. 5, wound drain 300 includes a porous insert 350 restrictively seated within the dome chamber. Insert 350 is composed of a fibrous or open-celled matrix of suitable materials that allows fluid flow therethrough, but lends internal structural support to the dome wall. Insert 350 is configured to restrictively seat within the dome chamber in a "press-fit" type connection similar to that of drain 100.

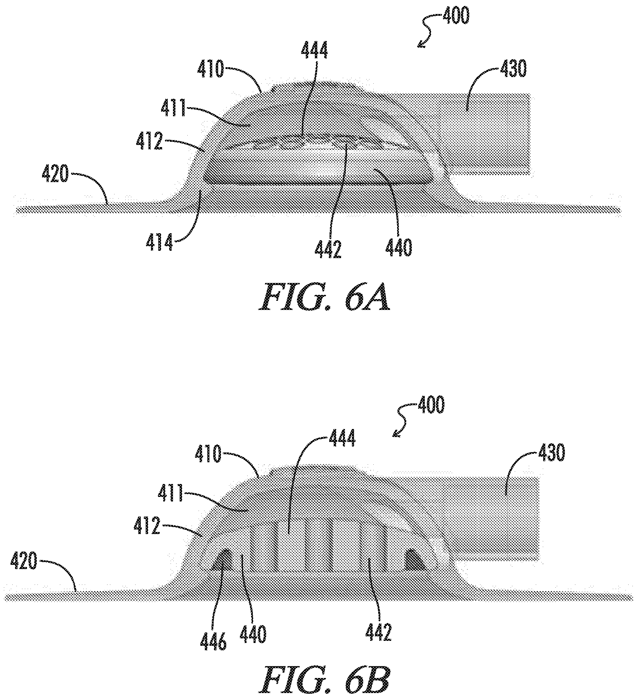

FIG. 6A depicts a side view of another embodiment of the wound drain of the present disclosure. Wound drain 400 has semi-spherical dome 410 integrally rising from flat annular flange 420 extending radially from the dome. Dome 410 has a dome wall 412 defining an interior dome chamber 411 with an open bottom. The dome further includes port connection 430 in fluid communication with dome interior 411. The dome 410 is shaped to have annular lip 414. Annular lip 414 holds a support structure, which in this embodiment is a grate 440. Grate 440 comprises a plurality of apertures providing fluid communication between the dome chamber 411 and a wound (not depicted). Grate 440 in some embodiments has a thickness that extends into the dome chamber. Grate 440 also may comprise a slightly domed or arcuate shape 444. FIG. 6B depicts a cross sectional side view of dome 400. Grate 440 may be shaped such that an annular cavity 446 extends upward towards dome 410. Wound drain 400 is formed, molded or otherwise made of a pliable material, suitable for surgical and medical use. The pliable material may be a material suitable for medical purposes, such as a silicon rubber or Arkema Pebax^{®} polymer. The pliable construction allows the drain to conform and contort so that the flange lies flat against the patient's skin, even over, around and into body creases and folds. The support structure, such as grate 440, is made of a material that is more rigid than the dome material. It should be noted that the dome shape has a generous radius to allow for more pliability and reduced pressure points on the wound.

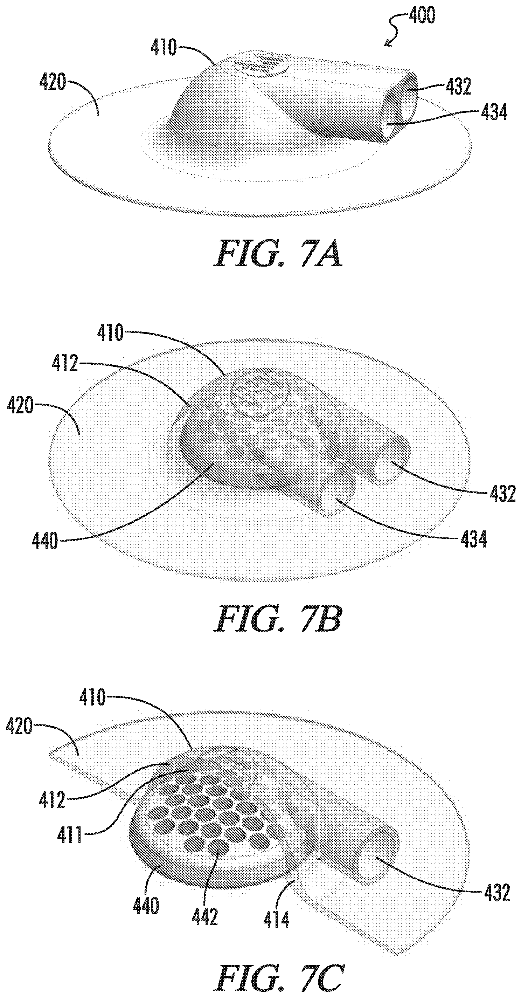

Turning to FIGS. 7A to 7C, an embodiment of a wound drain having a dual lumen is depicted. FIG. 7A depicts a perspective view of wound drain 400 having dome 410, flange 420 and first and second lumens 432 and 434, respectively, each in fluid communication with the dome interior 411. The first lumen provides connection to a drain line or negative pressure source (not depicted), while the second lumen advantageously provides a vent and/or an injection port for the introduction of fluids or gases. FIG. 7B is another perspective view depicting the interior of the dome 410 so that dome wall 412 and grate 440 can be seen. FIG. 7C is perspective partial cross sectional view of dome 400 depicting the apertures 442 of grate 440. FIG. 7C further shows dome interior chamber 411 and annular lip 414.

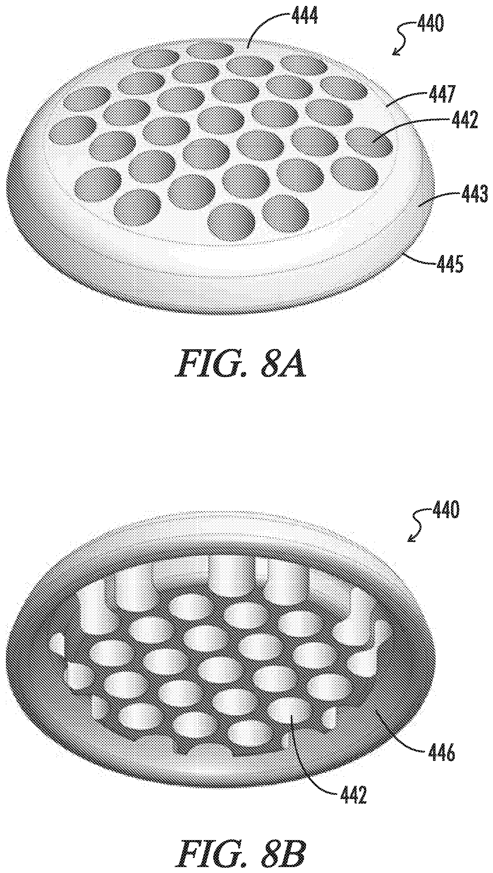

Turning to FIG. 8A, a top perspective view of grate 440 depicts a plurality of apertures 442, and comprises an arcuate shape 444. The apertures 442 may be of any size or shape suitable for allowing sufficient flow of wound exudate while preventing material from entering the lumen or lumens of the drain and also for manufacturing convenience. In this particular embodiment, apertures 442 are generally circular and arranged in a plurality of rows. The number of apertures can range from about 10 to about 100, about 10 to about 50, about 20 to about 50 or about 25 to about 35. Sidewall 443 of the grate generally curves inwardly, such that the radius of the bottom of the grate 445 is larger than the radius towards the top 447. FIG. 8B depicts a bottom perspective view of grate 440. Grate 440 may have a generally hollow annular ring 446.

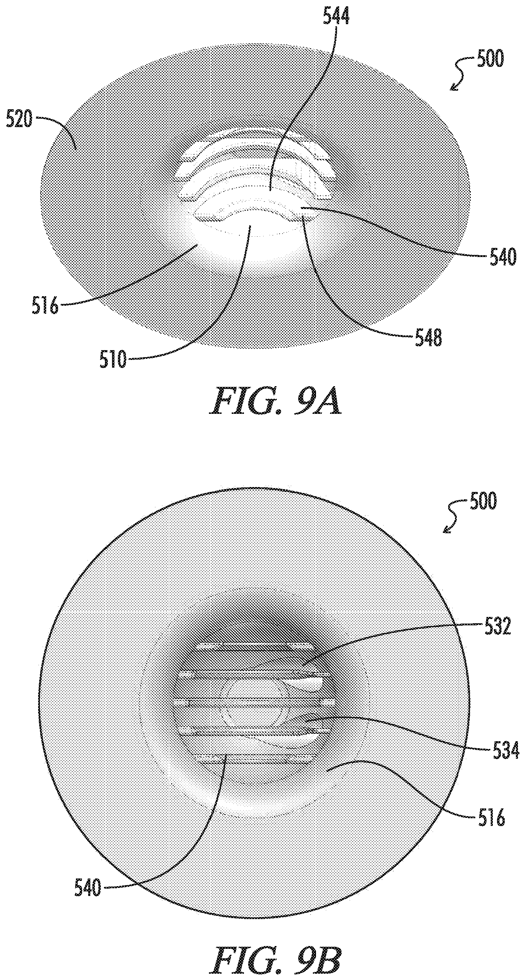

FIGS. 9A and 9B depict yet another embodiment of wound drain 500 having a dome 510 (depicted from inside the drain), flange 520 extending radially from the dome and curved portion 516 extending upwardly from the flange toward the dome 510. In this embodiment, drain 500 comprises a support structure of a plurality of arcuate ribs 540 integrally formed with curved portion 516. FIG. 9B depicts a perspective view from underneath the drain upward towards the dome. In this embodiment, dual lumens 532 and 534 are depicted.

Thus, although there have been described particular embodiments of the present invention of a new and useful wound drain it is not intended that such references be construed as limitations upon the scope of this invention which is as set forth in the following claims.

## Claims

1. A wound drain (100, 200, 300, 400) for a wound cavity, the drain comprising:
a dome (110, 210, 410, 510) having a chamber wall defining an internal dome chamber (111,411) with an open bottom;
an integral peripheral flange (120,220,420,520) extending radially from the dome;
a port connection (130,230,430) extending from the dome having a passage therethrough in fluid communication with the dome chamber; and
a support structure (140,240,440,540) within the dome chamber, the support structure comprising a plurality of openings for preventing foreign objects within the wound cavity from migrating into the dome chamber while allowing fluid flow from the wound cavity through the dome chamber and passage,
wherein the dome and flange are composed of a pliable material and the support structure is composed of a pliable material that is more rigid than the material of the dome and flange.

2. The drain of claim 1 wherein the support structure is a flat screen (140,240) affixed to the flange and underlying the dome chamber.

3. The drain of claim 1, wherein the support structure is a grate (440) comprising a plurality of apertures.

4. The drain of claim 3, wherein the apertures are disposed in a circular shape.

5. The drain of claims 1 or 2, wherein the support structure comprises a plurality of arcuate ribs (540).

6. The drain of any preceding claim wherein the chamber wall includes an annular lip (114) extending internally into the dome chamber adjacent the flange to form an annular seat (116) within the dome chamber.

7. The drain of claim 6, wherein the support structure comprises a flat screen fitted 4836-6721-4525.1 within the seat.

8. The drain of claim 6, wherein the support structure comprises a grate having a plurality of apertures (442) fitted within the seat.

9. The drain of any preceding claim, further comprising a second port connection (432,434), the second port connection being in fluid communication with the dome chamber.

10. The drain of claim 9, wherein the second port connection is configured to provide a vent and/or an injection port for the introduction of fluid or gas into the dome chamber.

11. The drain of claim 1, wherein the support structure comprises a porous insert (350) disposed within the dome chamber, and the porous insert comprises an open celled matrix material.

## Patentansprüche

1. Wunddrainage (100, 200, 300, 400) für eine Wundhöhle, wobei die Drainage umfasst:
eine Kuppel (110, 210, 410, 510) mit einer Kammerwand, die eine interne Kuppelkammer (111, 411) mit einem geöffneten Boden definiert,
einen integrierten Umfangsflansch (120, 220, 420, 520), der sich radial von der Kuppel erstreckt,
eine Anschlussverbindung (130, 230, 430), die sich von der Kuppel erstreckt und einen sich durch ihn erstreckenden Kanal in einer Fluidverbindung mit der Kuppelkammer aufweist, und
einen Halteaufbau (140, 240, 440, 540) in der Kuppelkammer, wobei der Halteaufbau eine Vielzahl von Öffnungen umfasst, die ein Migrieren von Fremdobjekten aus der Wundhöhle in die Kuppelkammer verhindern und gleichzeitig einen Fluidfluss von der Wundhöhle durch die Kuppelkammer und den Kanal erlauben,
wobei die Kuppel und der Flansch aus einem biegsamen Material bestehen und der Halteaufbau aus einem biegsamen Material, das steifer als das Material der Kuppel und des Flansches ist, besteht.

2. Drainage nach Anspruch 1, wobei der Halteaufbau ein flaches Sieb (140, 240) ist, das an dem Flansch fixiert und unter der Kuppelkammer angeordnet ist.

3. Drainage nach Anspruch 1, wobei der Halteaufbau ein Gitter (440) mit einer Vielzahl von Öffnungen ist.

4. Drainage nach Anspruch 3, wobei die Öffnungen in einer kreisrunden Form angeordnet sind.

5. Drainage nach Anspruch 1 oder 2, wobei der Halteaufbau eine Vielzahl von bogenförmigen Rippen (540) umfasst.

6. Drainage nach einem der vorstehenden Ansprüche, wobei die Kammerwand eine ringförmige Rippe (114) umfasst, die sich nach innen in die Kuppelwand in Nachbarschaft zu dem Flansch erstreckt, um einen ringförmigen Sitz (116) in der Kuppelkammer zu bilden.

7. Drainage nach Anspruch 6, wobei der Halteaufbau ein flaches Sieb, das in den Sitz gepasst ist, umfasst.

8. Drainage nach Anspruch 6, wobei der Halteaufbau ein Gitter mit einer Vielzahl von Öffnungen (442), das in den Sitz gepasst ist, umfasst.

9. Drainage nach einem der vorstehenden Ansprüche, das weiterhin eine zweite Anschlussverbindung (432, 434) umfasst, wobei die zweite Anschlussverbindung in einer Fluidverbindung mit der Kuppelkammer ist.

10. Drainage nach Anspruch 9, wobei die zweite Anschlussverbindung konfiguriert ist zum Vorsehen einer Öffnung und/oder eines Einführanschlusses für das Einführen eines Fluids oder Gases in die Kuppelkammer.

11. Drainage nach Anspruch 1, wobei der Halteaufbau einen in der Kuppelkammer angeordneten porösen Einsatz (350) umfasst und der poröse Einsatz ein offenzelliges Matrixmaterial umfasst.

## Revendications

1. Drain de plaie (100, 200, 300, 400) pour une cavité de plaie, le drain comprenant :
un dôme (110, 210, 410, 510) présentant une paroi de chambre définissant une chambre de dôme interne (111, 411) avec un fond ouvert ;
une collerette périphérique intégrale (120, 220, 420, 520) s'étendant radialement à partir du dôme ;
une liaison de port (130, 230, 430) s'étendant à partir du dôme et comportant un passage en communication fluidique avec la chambre de dôme ; et
une structure de support (140, 240, 440, 540) à l'intérieur de la chambre de dôme, la structure de support comprenant une pluralité d'ouvertures pour empêcher les corps étrangers à l'intérieur de la cavité de plaie de migrer dans la chambre de dôme tout en permettant un débit de fluide de la cavité de plaie à travers la chambre du dôme et le passage,
dans lequel le dôme et la collerette sont composés d'un matériau souple et la structure de support est composée d'un matériau souple plus rigide que le matériau du dôme et de la collerette.

2. Drain selon la revendication 1, dans lequel la structure de support est un écran plat (140, 240) fixé à la collerette et sous-jacent à la chambre de dôme.

3. Drain selon la revendication 1, dans lequel la structure de support est une grille (440) comprenant une pluralité d'ouvertures.

4. Drain selon la revendication 3, dans lequel les ouvertures sont disposées de manière circulaire.

5. Drain selon les revendications 1 ou 2, dans lequel la structure de support comprend une pluralité de nervures arquées (540).

6. Drain selon l'une quelconque revendication précédente, dans lequel la paroi de la chambre comporte une lèvre annulaire (114) s'étendant à l'intérieur de la chambre du dôme en étant adjacente à la collerette pour former un siège annulaire (116) à l'intérieur de la chambre de dôme.

7. Drain selon la revendication 6, dans lequel la structure de support comprend un écran plat installé à l'intérieur du siège.

8. Drain selon la revendication 6, dans lequel la structure de support comprend une grille présentant une pluralité d'ouvertures (442) installées à l'intérieur du siège.

9. Drain selon l'une quelconque revendication précédente, comprenant, en outre, une deuxième liaison de port (432, 434), la deuxième liaison de port étant en communication fluidique avec la chambre de dôme.

10. Drain selon la revendication 9, dans lequel la deuxième liaison de port est conçue pour fournir un évent et/ou un port d'injection pour l'introduction d'un fluide ou d'un gaz dans la chambre de dôme.

11. Drain selon la revendication 1, dans lequel la structure de support comprend un insert poreux (350) disposé dans la chambre de dôme, et l'insert poreux comprend un matériau matriciel à cellules ouvertes.
